(19) Europäisches Patentamt
European Patent Office
Office européen des brevets

(11) Veröffentlichungsnummer: **0 286 929**
**A2**

(12) # EUROPÄISCHE PATENTANMELDUNG

(21) Anmeldenummer: **88105333.4**

(22) Anmeldetag: **01.04.88**

(51) Int. Cl.4: **C07D 207/08 , C07D 405/12 , C07D 405/14 , C07D 401/12 , C07K 5/06 , C07K 5/08 , A61K 31/40 , A61K 31/44 , A61K 31/505 , A61K 37/02**

(30) Priorität: **11.04.87 DE 3712363**

(43) Veröffentlichungstag der Anmeldung:
**19.10.88 Patentblatt 88/42**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Henning, Rainer, Dr.**
**Rotenhofstrasse 31**
**D-6234 Hattersheim am Main(DE)**
Erfinder: **Hock, Franz, Dr.**
**Altstadt 19**
**D-6110 Dieburg(DE)**

(54) **Neue Pyrrolidin-Derivate, Verfahren zu ihrer Herstellung, sie enthaltende Mittel sowie deren Verwendung.**

(57) Die Erfindung betrifft Pyrrolidin-Derivate der allgemeinen Formel

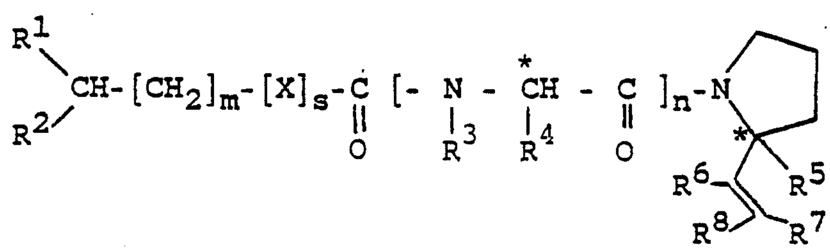

in welcher $R^1$, $R^2$, $R^3$, $R^4$, $R^5$, $R^6$, $R^7$ und $R^8$ die in der Beschreibung angegebenen Bedeutungen haben, X Sauerstoff, Imino oder N-Alkylimino bedeutet, m = 0-5 ist, n = 0-2 ist und s = 0 oder 1 ist, Verfahren zu ihrer Herstellung, sie enthaltende Mittel sowie deren Verwendung.

**EP 0 286 929 A2**

## Neue Pyrrolidin-Derivate, Verfahren zu ihrer Herstellung, sie enthaltende Mittel Sowie deren Verwendung

Die Erfindung betrifft Pyrrolidin-Derivate der allgemeinen Formel I

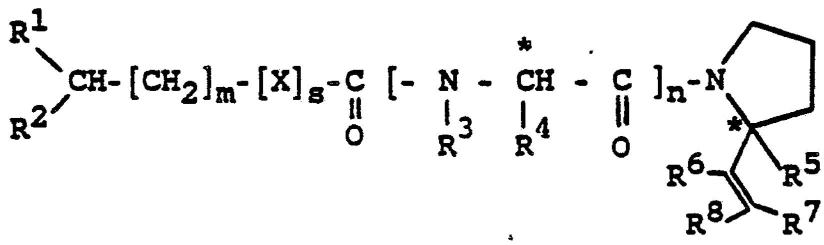

(I)

in welcher

$R^1$ Wasserstoff; $(C_1-C_6)$-Alkyl oder $(C_6-C_{12})$-Aryl, das gegebenenfalls durch einen, zwei oder drei gleiche oder verschiedene Reste aus der Reihe $(C_1-C_4)$-Alkyl, $(C_1-C_4)$-Alkoxy, Halogen, Nitro, Hydroxy, Amino, $(C_1-C_4)$-Alkylamino und Di-$(C_1-C_4)$-alkylamino, oder ein ($C_1$ oder $C_2$)-Alkylendioxy substituiert ist, bedeutet;

$R^2$ Wasserstoff; $(C_1-C_6)$-Alkyl; $(C_6-C_{12})$-Aryl; $(C_6-C_{12})$-Aryloxy; $(C_7-C_{13})$-Aroyl; Hydroxy oder $(C_1-C_4)$-Alkoxy bedeutet, wobei Aryl, Aryloxy und Aroyl jeweils gegebenenfalls durch einen, zwei oder drei gleiche oder verschiedene Reste aus der Reihe $(C_1-C_4)$-Alkyl, $(C_1-C_4)$-Alkoxy, Halogen, Nitro, Hydroxy, Amino, $(C_1-C_4)$-Alkylamino und Di-$(C_1-C_4)$-alkylamino, oder ein ($C_1$ oder $C_2$)-Alkylendioxy substituiert ist, bedeutet; oder

$R^1$ und $R^2$ zusammen für Benzyliden stehen, worin der Phenylring gegebenenfalls durch einen, zwei oder drei gleiche oder verschiedene Reste aus der Reihe $(C_1-C_4)$-Alkyl, $(C_1-C_4)$-Alkoxy, Halogen, Nitro, Hydroxy, Amino, $(C_1-C_4)$-Alkylamino, und Di-$(C_1-C_4)$-alkylamino, oder ein ($C_1$ oder $C_2$)-Alkylendioxy substituiert ist;

$R^3$ Wasserstoff; $(C_1-C_6)$-Alkyl; $(C_6-C_{12})$-Aryl-$(C_1-C_6)$-alkyl; $(C_5-C_9)$-Cycloalkyl;1-oder 2-Indanyl oder $\alpha$-oder $\beta$-1,2,3,4-Tetrahydronaphthyl bedeutet;

$R^4$ Wasserstoff; $(C_1-C_6)$-Alkyl, das durch Amino, $(C_1-C_6)$-Acylamino, insbesondere $(C_1-C_6)$-Alkanoylamino oder Boc-NH, oder Benzoylamino monosubstituiert sein kann; $(C_2-C_6)$-Alkenyl; $(C_5-C_9)$-Cycloalkyl; $(C_5-C_9)$-Cycloalkenyl; $(C_5-C_7)$-Cycloalkyl-$(C_1-C_4)$-alkyl; $(C_6-C_{12})$-Aryl oder teilhydriertes $(C_6-C_{12})$-Aryl, die jeweils durch einen, zwei oder drei gleiche oder verschiedene Reste aus der Reihe $(C_1-C_4)$-Alkyl, ($C_1$ oder $C_2$)-Alkoxy und Halogen substituiert sein können; $(C_6-C_{12})$-Aryl-$(C_1-C_4)$-alkyl oder $(C_7-C_{13})$-Aroyl-($C_1$ oder $C_2$)-alkyl, die beide wie vorstehend definiert im Arylrest substituiert sein können; einen mono-bzw. bicyclischen Heterocyclen-Rest mit 5 bis 7 bzw. 8 bis 10 Ringatomen, wovon 1 bis 9 Ringatome Kohlenstoffatome und 1 bis 2 Ringatome Schwefel-oder Sauerstoffatome und/oder wovon 1 bis 4 Ringatome Stickstoffatome darstellen; oder, falls von vorstehenden Definitionen noch nicht umfaßt, die gegebenenfalls geschützte Seitenkette einer natürlich vorkommenden $\alpha$-Aminosäure bedeutet; oder

$R^3$ und $R^4$ zusammen für $-[CH_2]_p$-stehen, worin $p = 3$, 4 oder 5 ist und worin eine Methylengruppe durch S oder O ersetzt sein kann;

$R^5$ Wasserstoff; $(C_1-C_6)$-Alkyl; $(C_6-C_{12})$-Aryl-$(C_1-C_5)$-alkyl, das im Arylteil gegebenenfalls durch einen, zwei oder drei gleiche oder verschiedene Reste aus der Reihe $(C_1-C_4)$-Alkyl, $(C_1-C_4)$-Alkoxy, Halogen, Nitro, Hydroxy, Amino, $(C_1-C_4)$-Alkylamino und Di-$(C_1-C_4)$-alkylamino, oder ein ($C_1$ oder $C_2$)-Alkylendioxy substituiert ist, bedeutet;

$R^6$ Wasserstoff; $(C_1-C_6)$-Alkyl oder $(C_6-C_{12})$-Aryl, das gegebenenfalls durch einen oder zwei gleiche oder verschiedene Reste aus der Reihe $(C_1-C_4)$-Alkyl, ($C_1$ oder $C_2$)-Alkoxy und Halogen, oder ein ($C_1$ oder $C_2$)-Alkylendioxy substituiert ist, bedeutet;

$R^7$ Wasserstoff; $(C_1-C_6)$-Alkyl; Cyano; $(C_7-C_{13})$-Aroyl, das gegebenenfalls durch einen oder zwei gleiche oder verschiedene Reste aus der Reihe $(C_1-C_4)$-Alkyl, ($C_1$ oder $C_2$)-Alkoxy, Halogen und Nitro, oder ein ($C_1$ oder $C_2$)-Alkylendioxy substituiert ist; $(C_1-C_6)$-Alkoxycarbonyl oder $(C_1-C_8)$-Alkanoyl bedeutet;

$R^8$ Cyano; $(C_7-C_{13})$-Aroyl oder $(C_6-C_{12}$-Aryl-$(C_2-C_4)$-alkanoyl bedeutet, wobei jeweils Aryl durch einen, zwei oder drei gleiche oder verschiedene Reste aus der Reihe $(C_1-C_4)$-Alkyl, $(C_1-C_4)$-Alkoxy, Halogen, Nitro und Hydroxy oder ein ($C_1$ oder $C_2$)-Alkylendioxy substituiert sein kann; oder $(C_1-C_6)$-Alkanoyl; $(C_1-C_6)$-Alkoxycarbonyl; Benzyloxycarbonyl oder einen Rest der Formel

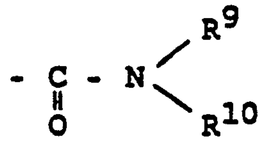

bedeutet;

$R^9$ und $R^{10}$ gleich oder verschieden sind und Wasserstoff; $(C_1\text{-}C_8)$-Alkyl; $(C_6\text{-}C_{12})$-Aryl oder $(C_6\text{-}C_{12})$-Aryl-$(C_1\text{-}C_8)$-alkyl, worin jeweils Aryl gegebenenfalls durch einen, zwei oder drei gleiche oder verschiedene Reste aus der Reihe $(C_1\text{-}C_4)$-Alkyl, $(C_1\text{-}C_4)$-Alkoxy, Halogen und Hydroxy oder ein ($C_1$ oder $C_2$)-Alkylendioxy substituiert ist; oder $(C_5\text{-}C_9)$-Cycloalkyl bedeuten; oder

$R^9$ und $R^{10}$ zusammen mit dem diese tragenden N-Atom einen 5-bis 10-gliedrigen heterocyclischen Ring bilden, der als Ringatome 2-9 Kohlenstoffatome und gegebenenfalls 1 oder 2 weitere gleiche oder verschiedene Heteroatome aus der Reihe Sauerstoff, Schwefel und Stickstoff enthält und der gegebenenfalls durch einen Rest aus der Reihe $(C_1\text{-}C_8)$-Aryl, Phenyl, Phenyl-$(C_1\text{-}C_4)$-alkyl, Benzoyl, Phenyl-$(C_2\text{-}C_4)$-alkanoyl, 2-oder 3-Furoyl, 2-, 3-oder 4-Pyridyl und 2-, 4-oder 5-Pyrimidinyl substituiert ist, worin Phenyl, Phenylalkyl, Benzoyl und Phenylalkanoyl ihrerseits jeweils im Phenylrest durch einen, zwei oder drei gleiche oder verschiedene Reste aus der Reihe $(C_1\text{-}C_4)$-Alkyl, $(C_1\text{-}C_4)$-Alkoxy, Halogen, Hydroxy, Cyano und Nitro, oder ein ($C_1$ oder $C_2$)-Alkylendioxy substituiert sein können;

X Sauerstoff, Imino oder N-$(C_1\text{-}C_8)$-Alkylimino bedeutet;

m = 0, 1, 2, 3, 4 oder 5 ist;

n = 0, 1 oder 2 ist und

s = 0 oder 1 ist,

sowie deren physiologisch verträgliche Salze, falls solche gebildet werden können.

Alkyl kann geradkettig oder verzweigt sein und ist beispielsweise Methyl, Ethyl, Propyl, Isopropyl, Butyl, Isobutyl, sec.-Butyl, tert.-Butyl, Pentyl, Isopentyl, Neopentyl, sec.-Pentyl, tert.-Pentyl, Hexyl, Isohexyl, Heptyl oder Octyl. Entsprechendes gilt für davon abgeleitete Reste wie Alkoxy, Alkylamino, Dialkylamino, Alkanoyl, Alkoxycarbonyl und Aralkyl.

Aryl ist beispielsweise Phenyl, α-oder β-Naphthyl, 2-, 3-oder 4-Biphenyl; bevorzugt ist Phenyl. Entsprechendes gilt für davon abgeleitete Reste wie Aryloxy, Aralkyl, Aryl, Aroyl und Arylalkanoyl.

Halogen ist Fluor, Brom oder Jod; bevorzugt sind Fluor, Chlor und Brom.

Unter $R^4$ in der Bedeutung eines mono-bzw. bicyclischen Heterocyclen-Resten mit 5 bis 7 bzw. 8 bis 10 Ringatomen, wovon 1 bis 9 Ringatome Kohlenstoffatome und 1 bis 2 Ringatome Schwefel-oder Sauerstoffatome und/oder wovon 1 bis 4 Ringatome Stickstoffatome darstellen, wird beispielsweise Thienyl, Benzo[b]thienyl, Furyl, Pyranyl, Benzofuryl, Pyrrolyl, Imidazolyl, Pyrazolyl, Pyridyl, Pyrimidinyl, Pyridazinyl, Indazolyl, Isoindolyl, Indolyl, Purinyl, Chinolizinyl, Isochinolinyl, Phthalazinyl, Naphthyridinyl, Chinozalinyl, Chinazolyl, Cinnolinyl, Pteridinyl, Oxazolyl, Isoxazolyl, Thiazolyl oder Isothiazolyl verstanden. Diese Reste können auch teilweise oder vollständig hydriert sein.

$R^9$ und $R^{10}$ können zusammen mit dem diese tragenden N-Atom einen 5-bis 10-gliedrigen, vorzugsweise 5-bis 7-gliedrigen heterocyclischen Ring bilden, der als Ringatome 2-9 Kohlenstoffatome und gegebenenfalls 1 oder 2 weitere gleiche oder verschiedene Heteroatome aus der Reihe Sauerstoff, Schwefel und Stickstoff enthält und der gegebenenfalls in der oben angegebenen Weise monosubstituiert ist. Dieser Heterocyclenrest kann gesättigt oder ungesättigt, mono-oder bicyclisch sein. Bevorzugt sind jedoch monocyclische gesättigte Reste, wie z.B. Piperidino, Pyrrolidino, 1-Azepinyl, Morpholino, Piperazino oder Homopiperazino.

Verbindungen der Formel I besitzen chirale C-Atome. Sowohl die R-als auch die S-Konfiguration an allen Asymmetriezentren sind Gegenstand der Erfindung. Die Verbindungen der Formel I können daher als optische Isomere, als Diasteromere, als Racemate oder als Gemische derselben vorliegen. Bevorzugt sind jedoch die Verbindungen der Formel I, in denen die mit einem Stern (*) markierten C-Atome S-Konfiguration aufweisen. Falls $R^4$ für die Seitenkette von Cystein steht, wird jedoch die R-Konfiguration dieses Zentrums bevorzugt.

Natürlich vorkommende α-Aminosäuren,wie beispielsweise Ala, Val, Leu, Ile, Phe, Abu, C-Ph-Gly, His, Trp, Lys, Orn, Dab, Dap, Daap, Dapi, Arg, Cit, Glu, Gln, Asp, Asn, Cys, Met, Hyl, Ser, Thr.und Tyr sind z.B. in Ann. Rev. Biochem. 38 [1969] 137-158 und FEBS Letters 64 [1976] 29-35 beschrieben.

Falls $R^4$ für eine geschützte Seitenkette einer natürlich vorkommenden α-Aminosäure steht, wie z.B. geschütztes Ser, Thr, Asp, Asn, Glu, Gln, Arg, Lys, Hyl, Cys, Orn, Cit, Tyr, Trp oder His, sind als Schutzgruppen die in der Peptidchemie üblichen Gruppen bevorzugt (vgl. z.B. T.W. Greene, "Protective

Groups in Organic Synthesis, New York, 1981 oder Bodanszky, Bodanszky, "Principles bzw. Practice of Peptide Synthesis", Berlin, 1984). Im Falle, daß $R^4$ eine geschützte Lysin-Seitenkette bedeutet, werden die bekannten Aminoschutzgruppen, insbesondere aber $(C_1-C_6)$-Alkanoyl bevorzugt. Falls $R^4$ eine geschützte Tyrosin-Seitenkette bedeutet, wird die Ether-Schutzgruppe am Sauerstoff, insbesondere $(C_1-C_6)$-Alkyl, bevorzugt; besonders bevorzugte Schutzgruppen sind Methyl und Ethyl.

Als Salze kommen insbesondere Alkali-oder Erdalkalisalze, Salze mit physiologisch verträglichen Aminen und Salze mit anorganischen oder organischen Säuren wie z.B. HCl, HBr, $H_2SO_4$, Maleinsäure, Fumarsäure in Frage.

Bevorzugt sind Verbindungen der Formel I, in welcher

$R^1$ Wasserstoff; $(C_1-C_4)$-Alkyl oder Phenyl, das gegebenenfalls durch einen oder zwei gleiche oder verschiedene Reste aus der Reihe Methyl, Ethyl, Methoxy, Fluor, Chlor, Brom, Nitro, Hydroxy, Amino, Methylamino und Dimethylamino, oder drei Methoxy, oder ein Methylendioxy substituiert ist, bedeutet;

$R^2$ Wasserstoff; Phenyl; Phenoxy; Benzoyl; Hydroxy oder Methoxy bedeutet, wobei Phenyl, Phenoxy und Benzoyl jeweils gegebenenfalls durch einen oder zwei gleiche oder verschiedene Reste aus der Reihe Methyl, Ethyl, Fluor, Chlor, Brom, Nitro, Hydroxy, Amino, Methylamino und Dimethylamino, oder ein Methylendioxy substituiert ist; oder

$R^1$ und $R^2$ zusammen für Benzyliden stehen, worin der Phenylrest gegebenenfalls durch einen oder zwei gleiche oder verschiedene Reste aus der Reihe Methyl, Ethyl, Methoxy, Fluor, Chlor, Brom und Hydroxy, oder drei Methoxy, oder ein Methylendioxy substituiert ist;

$R^3$ Wasserstoff; $(C_1-C_4)$-Alkyl; Benzyl; Phenethyl; Cyclopentyl; Cyclohexyl oder 2-Indanyl bedeutet;

$R^4$ Wasserstoff oder die gegebenenfalls geschützte Seitenkette einer natürlich vorkommenden α-Aminosäure bedeutet; oder

$R^3$ und $R^4$ zusammen für einen Rest $-[CH_2]_p$-stehen, der wie im Anspruch 1 definiert ist und worin $p = 3$ oder 4 ist;

$R^5$ Wasserstoff; Methyl; Ethyl; Benzyl oder Phenethyl, welche jeweils im Phenylrest gegebenenfalls durch einen, zwei oder drei gleiche oder verschiedene Reste aus der Reihe Methoxy, Fluor, Chlor und Brom, oder ein Methylendioxy substituiert sind, bedeutet;

$R^6$ Wasserstoff; $(C_1-C_4)$-Alkyl; Phenyl; o-, m-oder p-Methoxyphenyl; 2,3-, 2,4-, 2,5-, 2,6-, 3,4-oder 3,5-Dimethoxyphenyl; o-, m-oder p-Tolyl; 2,3-, 2,4-, 2,5-, 2,6-, 3,4-oder 3,5-Xylyl; o-, m-oder p-Fluorphenyl; o-, m-oder p-Chlorphenyl oder 2,3-oder 3,4-Methylendioxyphenyl bedeutet;

$R^7$ Wasserstoff; $(C_1-C_4)$-Alkyl; Cyano; Benzoyl, das gegebenenfalls durch einen oder zwei gleiche oder verschiedene Reste aus der Reihe Methyl, Methoxy, Fluor, Chlor, Brom und Nitro, oder ein Methylendioxy substituiert ist; $(C_1-C_4)$-Alkoxycarbonyl oder $(C_1-C_5)$-Alkanoyl bedeutet;

$R^8$ Cyano; Benzoyl, das gegebenenfalls durch einen oder zwei gleiche oder verschiedene Reste aus der Reihe Methyl, Methoxy, Fluor, Chlor und Brom, drei Methoxy, oder ein Methylendioxy substituiert ist; $(C_1-C_4)$-Alkanoyl; $(C_1-C_4)$-Alkoxycarbonyl oder einen Rest der Formel

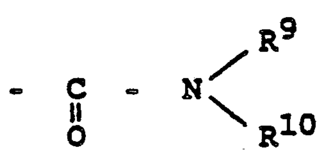

bedeutet;

$R^9$ und $R^{10}$ gleich oder verschieden sind und Wasserstoff; $(C_1-C_6)$-Alkyl; Phenyl oder Phenyl-$(C_1-C_4)$-alkyl, worin der Phenylrest jeweils durch einen oder zwei gleiche oder verschiedene Reste aus der Reihe Methyl, Ethyl, Methoxy, Fluor, Chlor, Brom und Hydroxy, drei Methoxy oder ein Methylendioxy substituiert sein kann; Cyclopentyl; Cyclohexyl oder Cycloheptyl bedeuten; oder $R^9$ und $R^{10}$ zusammen mit dem diese tragenden N-Atom einen 5-bis 7-gliedrigen heterocyclischen Ring bilden, der als Ringatome 2-6 Kohlenstoffatome und gegebenenfalls 1 oder 2 weitere gleiche oder verschiedene Heteroatome aus der Reihe Sauerstoff, Schwefel und Stickstoff enthält und der gegebenenfalls durch einen Rest aus der Reihe $(C_1-C_4)$-Alkyl, Phenyl, Phenyl-$(C_1-C_4)$-alkyl, Benzoyl, 2-oder 3-Furoyl, 2-, 3-oder 4-Pyridyl und 2-, 4-oder 5-Pyrimidinyl substituiert ist, worin Phenyl, Phenylalkyl und Benzoyl ihrerseits jeweils im Phenylrest durch einen oder zwei gleiche oder verschiedene Reste aus der Reihe Methyl, Methoxy, Fluor, Chlor, Brom, Cyano, Nitro und Hydroxy, drei Methoxy oder ein Methylendioxy substituiert sein können;

X Sauerstoff bedeutet;

m = 0, 1, 2, 3, 4 oder 5 ist;

n = 0 oder 1 ist und
s = 0 oder 1 ist.

Besonders bevorzugt sind Verbindungen der Formel I, worin

$R^1$ Wasserstoff; Methyl; Ethyl; Propyl; Isopropyl; Phenyl; o-, m-oder p-Tolyl; o-, m-oder p-Chlorphenyl; o-, m-oder p-Fluorphenyl; o-, m-oder p-Methoxyphenyl oder 2,3-, 2,4-, 2,5-, 2,6-, 3,4-oder 3,5-Dimethoxyphenyl bedeutet;

$R^2$ Wasserstoff; Phenyl; o-, m-oder p-Tolyl; o-, m-oder p-Chlorphenyl; o-, m-oder p-Fluorphenyl; o-, m-oder p-Methoxyphenyl; 2,3-, 2,4-, 2,5-, 2,6-, 3,4-oder 3,5-Dimethoxyphenyl; Phenoxy; o-, m-oder p-Tolyloxy; o-, m-oder p-Chlorphenoxy; o-, m-oder p-Fluorphenoxy; o-, m-oder p-Methoxyphenoxy; 2,3-, 2,4-, 2,5-, 2,6-, 3,4-oder 3,5-Dimethoxyphenoxy; Benzoyl; o-, m-oder p-Toluoyl; o-, m-oder p-Chlorbenzoyl; o-, m-oder p-Fluobenzoyl; o-, m-oder p-Methoxybenzoyl; 2,3-, 2,4-, 2,5-, 2,6-, 3,4-oder 3,5-Dimethoxybenzoyl; Hydroxy oder Methoxy bedeutet; oder

$R^1$ und $R^2$ zusammen für Benzyliden; o-, m-oder p-Methylbenzyliden; o-, m-oder p-Methoxybenzyliden oder 2,3-, 2,4-, 2,5-, 2,6-, 3,4-oder 3,5-Dimethoxybenzyliden stehen;

$R^3$ Wasserstoff, Methyl oder Benzyl bedeutet;

$R^4$ Wasserstoff; Methyl; n-Propyl; Isopropyl; n-Butyl; Isobutyl; sec. Butyl oder Benzyl bedeutet; oder

$R^3$ und $R^4$ zusammen für $-[CH_2]_3$-stehen;

$R^5$ Wasserstoff oder Methyl bedeutet;

$R^6$ Wasserstoff oder Methyl bedeutet;

$R^7$ Wasserstoff; Methyl; Cyano; Benzoyl; o-, m-oder p-Methoxybenzoyl; o-, m-oder p-Chlorbenzoyl; 2,3-, 2,4-, 2,5-, 2,6-, 3,4-oder 3,5-Dimethoxybenzoyl; Acetyl; Methoxycarbonyl oder Ethoxycarbonyl bedeutet;

$R_8$ Cyano; Formyl; Benzoyl; o-, m-oder p-Methoxybenzoyl; o-, m-oder p-Chlorbenzoyl; 2,3-, 2,4-, 2,5-, 2,6-, 3,4-oder 3,5-Dimethoxybenzoyl; Acetyl; Methoxycarbonyl; Ethoxycarbonyl oder einen Rest der Formel

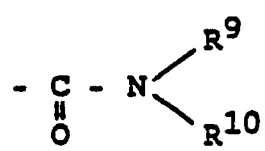

bedeutet;

$R^9$ und $R^{10}$ gleich oder verschieden sind und Wasserstoff; $(C_1-C_6)$-Alkyl; Phenyl oder Phenyl-$(C_1-C_4)$-alkyl, worin der Phenylrest jeweils durch einen oder zwei gleiche oder verschiedene Reste aus der Reihe Methyl, Methoxy, Fluor und Chlor, drei Methoxy, oder ein Methylendioxy substituiert sein kann; Cyclopentyl oder Cyclohexyl bedeutet; oder

$R^9$ und $R^{10}$ zusammen mit dem diese tragenden N-Atom einen 5-bis 7-gliedrigen heterocyclischen Ring bilden, der als Ringatome 2-6 Kohlenstoffatome und gegebenenfalls 1 oder 2 weitere gleiche oder verschiedene Heteroatome aus der Reihe Sauerstoff, Schwefel und Stickstoff enthält und der gegebenenfalls durch Methyl, Phenyl, Phenyl-$(C_1-C_4)$-alkyl, Benzoyl, Pyridyl oder Pyrimidinyl monosubstituiert ist, worin Phenyl, Phenylalkyl und Benzoyl ihrerseits jeweils im Phenylrest durch ein oder zwei gleiche oder verschiedene Reste aus der Reihe Methyl, Methoxy, Fluor, Chlor, Cyano und Nitro, oder drei Methoxy substituiert sein können;

X Sauerstoff bedeutet;

m = 0, 1, 2, 3, 4 oder 5 ist;
n = 0 oder 1 ist und
s = 0 oder 1 ist.

Insbesondere bevorzugt sind solche Verbindungen der Formel I, in welcher

$R^2$ Wasserstoff bedeutet;

$R^3$ und $R^4$ zusammen für $-[CH_2]_3$-stehen;

$R^5$ Wasserstoff bedeutet;

$R^6$ Wasserstoff bedeutet;

$R^7$ Wasserstoff bedeutet;

X Sauerstoff bedeutet;

m = 1, 2, 3 oder 4 ist;
n = 1 ist und
s = 0 oder 1 ist,

sowie solche Verbindungen der Formel I, in welcher

$R^5$ Wasserstoff bedeutet;
$R^6$ Wasserstoff bedeutet;
$R^7$ Wasserstoff bedeutet;
X Sauerstoff bedeutet;
m = 1, 2, 3 oder 4 ist;
n = 0 ist und
s = 0 oder 1 ist.

Die Erfindung betrifft auch ein Verfahren zur Herstellung von Verbindungen der Formel I, das dadurch gekennzeichnet ist, daß man

a) eine Verbindung der Formel II

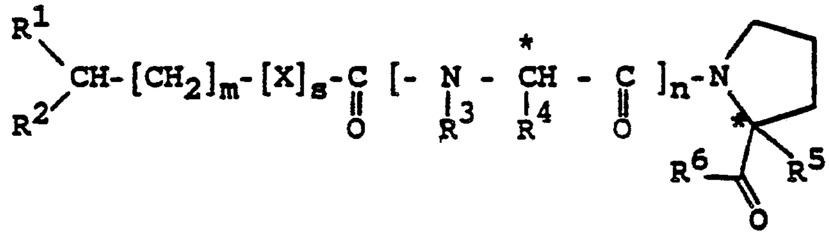

(II)

in welcher $R^1$, $R^2$, $R^3$, $R^4$, $R^5$ und $R^6$, X, m, n und s die gleiche Bedeutung wie in Formel I haben, in Gegenwart einer Base umsetzt mit einer Verbindung der Formel III,

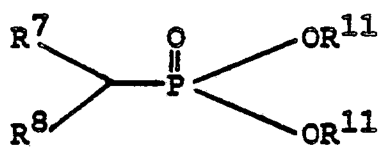

(III)

in welcher $R^7$ und $R^8$ die gleiche Bedeutung wie in Formel I haben und $R^{11}$ für $(C_1-C_4)$-Alkyl, Phenyl oder Phenyl-$(C_1-C_4)$-alkyl steht; oder

b) eine Verbindung der unter (a) definierten Formel II umsetzt mit einer Verbindung der Formel IV

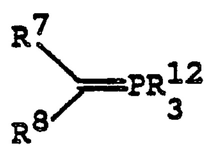

(IV)

in welcher $R^7$ und $R^8$ die gleiche Bedeutung wie in Formel I haben und $R^{12}$ für Phenyl, $(C_1-C_6)$-Alkylphenyl oder Di-$(C_1-C_6)$-alkylphenyl steht; oder

c) zur Herstellung einer Verbindung der Formel I, worin $R^8$ für einen Rest der Formel

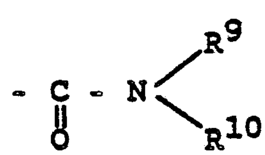

steht,

$c_1$) eine Verbindung der Formel I, in welcher $R^8$ $(C_1-C_6)$-Alkoxycarbonyl bedeutet und die übrigen Reste und Variablen wie oben definiert sind, umsetzt mit einer Verbindung der Formel V

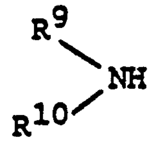

(V)

in welcher $R^9$ und $R^{10}$ die gleiche Bedeutung wie in Formel I haben; oder

$c_2$) eine Verbindung der Formel I, in welcher $R^8$ Carboxy bedeutet und die übrigen Reste und Variablen wie oben definiert sind, nach deren Umwandlung in ein aktiviertes Derivat mit einer Verbindung der unter ($c_1$) definierten Formel V umsetzt und die nach (a), (b) oder (c) erhaltenen Verbindungen gegebenenfalls in ihre physiologisch verträglichen Salze überführt.

Die Umsetzung gemäß Verfahrensvariante (a) (vgl. hierzu Chem. Revs. 74 [1974] 87) führt man in einem geeigneten organischen Lösungsmittel wie einem Ether (z.B. Diethylether, THF, Glyme, Diglyme), Kohlenwasserstoff (z.B. Cyclohexan, Toluol, Xylol) oder Amid (z.B. DMF, DMA) in Gegenwart einer Base wie einem Alkalialkoholat, Alkalihydrid, Alkalihydroxid, Alkaliamid, 1,5-Diazabicyclo-(4,3,0)-5-nonen, 1,8-Diazabicyclo(5,4,0)-7-undecen oder eine Lithiumdialkylamid bei -80°C bis +50°C, vorzugsweise bei -40°C bis +30°C durch.

Verfährt man nach Verfahrensvariante (b) (vgl. hierzu Accts. Chem. Res. 7 [1974] 6 und 85), so verwendet man als Reaktionsmedium ein aprotisches, organisches Lösungsmittel wie einen Kohlenwasserstoff (z.B. Benzol, Toluol, Xylol, Tetrahydronaphthalin), einen chlorierten Kohlenwasserstoff (z.B. Dichlormethan, Dichlorethan), ein Amid (wie DMF, DMA) oder DMSO bei einer Reaktionstemperatur von 0°C bis zum Siedepunkt des Reaktionsgemisches, vorzugsweise von 40°C bis zum Siedpunkt des Reaktionsgemisches.

Die Umamidierung gemäß Verfahrensvariante ($c_1$) wird in einem polaren protischen oder aprotischen Lösungsmittel wie eine, niederen Alkohol (z.B. Ethanol, n-Butanol), DMSO oder DMF bei 0°C bis zum Siedepunkt des Reaktionsgemisches, vorzugsweise bei 30°C bis zum Siedepunkt des Reaktionsgemisches oder ohne Lösungsmittel bei 0 bis 100°C durchgeführt.

Bei Verfahrensvariante ($c_2$) wird eine Verbindung der Formel I ($R^8$ = COOH) in ihr aktiviertes Derivat wie Säurechlorid, Anhydrid oder Aktivester (vgl. Schröder, Lübke "The Peptides", Volume I, New York, 1965, Seiten 77-128) überführt und in einem geeigneten organischen Lösungsmittel vorzugsweise aus der Reihe der bei Variante ($c_1$) genannten Lösungsmittel bei einer Temperatur zwischen -20°C und dem Siedepunkt des Reaktionsgemisches vorzugsweise zwischen 0°C und dem Siedepunkt des Reaktionsgemisches weiter umgesetzt.

Verbindungen der Formel II sind entweder aus der Literatur bekannt oder können in Analgie zu bekannten Verfahren hergestellt werden; einige Vertreter sind beispielsweise in europäischen Patentanmeldungen EP-A 172458 und EP-A 207742 und der japanischen Patentanmeldung 1183-297 sowie in Life Sci. 33, 2149 (1983) beschrieben.

Die erfindungsgemäßen Verbindungen der Formel I sind Hemmstoffe der Prolyl-endo-peptidase (EC 3.4. 21.26). Von diesem Enzym ist bekannt, daß es Neuropeptide wie Substanz P, Neurotensin, LHRH, TRH, Vasopressin sowie Angiotensin II abbaut (Life Sci. 33, 2149 (1983)). Diese Neuropeptide sind mit wichtigen Funktionen im Zentralnervensystem assoziiert. Durch Hemmung ihres Abbaus mittels Hemmung der Prolyl-endo-peptidase werden durch Verbindungen der Formel I verschiedenartige Wirkungen im ZNS ausgelöst, insbesondere anti-amnestische, antipsychotische, anxiolytische und antidepressive Wirkungen.

Verbindungen der Formel I eignen sich daher zur Behandlung verschiedener Erkrankungen des Zentralnervensystems, insbesondere als Nootropika und Antipsychotika bei Warmblütern, vorzugsweise beim Menschen. Die Anwendung der erfindungsgemäßen Verbindungen kann intravenös, subkutan oder peroral für sich allein oder im Kombination mit anderen ZNS-wirksamen Substanzen erfolgen.

Die Dosierung liegt je nach Art und Schwere der zu behandelnden Erkrankung bei 0,1-100 mg/kg/Tag, insbesondere bei 1-10 mg/kg/Tag. Sie kann in schweren Fällen auch erhöht werden, da toxische Eigenschaften bisher nicht beobachtet wurden.

Die erfindungsgemäßen Verbindungen können oral oder parenteral in entsprechender pharmazeutischer Zubereitung verabreicht werden. Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesiumcarbonat, Kaliumphosphat, Milchzucker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche

und tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanz wie Lösungsvermittler, Emulgatoren, oder weitere Hilfsstoffe in Lösung, Suspension oder Emulsion gebracht. Als Lösungsmittel für die neuen aktiven Verbindungen und die entsprechenden physiologisch verträglichen Salze kommen z.B. in Frage: Wasser, physiologische Kochsalzlösungen oder Alkohole, z.B. Ethanol, Propandiol, oder Glycerin, daneben auch Zuckerlösungen wie Glucose-oder Mannitlösungen, oder auch eine Mischung aus den verschiedenen genannten Lösungsmitteln.

Die folgenden Beispiele sollen die Erfindung erläutern, ohne sie auf die genannten Verbindungen zu beschränken.

Beispiel 1

3-[N-(N-Benzyloxycarbonyl-S-prolyl)-pyrrolidin-2-S-yl] acrylsäure-ethylester

0,61 g (2,7 mmol) Triethylphosphoniumacetat werden zu einer Suspension von 0,33 g Kalium-tert.butylat in 25 ml THF bei -78°C gegeben. Nach 10 Minuten bei -78°C wird auf Raumtemperatur aufgewärmt, dann nach 15 Minuten wieder auf -78°C abgekühlt und 0,9 g (2,7 mmol) n-Benzyloxycarbonyl-S-prolyl-S-prolinol zugefügt. Die Lösung wird langsam auf Raumtemperatur aufgewärmt und noch 3 Stunden nachgerührt. Nach Verdünnen mit Wasser wird mit Essigester extrahiert, mit $Na_2SO_4$ getrocknet und eingeengt. Chromatographie an Kieselgel (Essigester/Cyclohexan 2:1, dann Essigester) ergibt 1 g der Titelverbindung als farbloses, zähes Öl.

$[\alpha]_D^{22}$ : -55,3° (c = 1,1, Essigester)

$^1$H-NMR ($CDCl_3$)

$\delta$ = 7,9-7,7 (m, 5H); 6,95-6,7 (m, 1H); 5,9-5,65 (m, 1H); 5,3-4,9 (m, 2H); 4,9-4,8 + 4,7-4,6 (m, 1H); 4,55-4,4 (m, 1H); 4,3-4,1 (m, 3H); 3,9-3,3 (m, 4H); 2,3-1,7 (m, 6H); 1,7-1,5 (m, 2H); 1,4-1,2 (m, 3H) ppm.

MS: (m/z): 400 ($M^+$ + 1 100 %) (chemische Ionisation)

Beispiel 2

3-[N-(4-Phenyl-butyryl)-pyrrolidin-2-S-yl]-acrylsäure-methylester

1,1 g (6 mmol) Trimethylphosphoniumacetat werden in 20 ml THF gelöst und bei -30°C mit 0,67 g Kalium-tert.butylat versetzt. Nach 30 Minuten bei Raumtemperatur werden bei -78°C 1,1 g N-(4-Phenylbutyryl)-S-prolinal zugefügt. Nach langsamen Aufwärmen auf Raumtemperatur und 30 minütigem Rühren wird mit Wasser verdünnt und mit Essigester extrahiert. Die vereinigten Extrakte werden mit gesättigter wäßriger Kochsalzlösung gewaschen, über Natriumsulfat getrocknet und eingeengt. Chromatographie an Kieselgel (Essigester/Cyclohexan 2:1) ergibt 0.55 g der Titelverbindung als farbloses Öl.

$[\alpha]_D^{23}$ = -43,7° (c = 1, $CH_2Cl_2$);

$^1$H-NMR ($CDCl_3$):

7,2 (s, 5H); 7,05-6,6 (m, 1H); 6,1-5,6 (m, 1H); 4,9-4,2 (m, 1H); 3,73 + 3,7 (2S, 3H); 3,6-3,2 (m, 2H); 2,9-1,4 (m, 10H) ppm.

MS (m/z): 302 ($M^+$ + 1, 100 %), (chem. Ionisation)

Beispiel 3

3-[N-(4-Phenyl-butyryl)-pyrrolidin-2-S-yl]-acrylsäure

1 g 3-[N-(4-Phenyl-butyryl)-pyrrolidin-2-S-yl]-acrylsäuremethylester werden in 10 ml Methanol gelöst und mit 4 ml wäßriger 1N NaOH versetzt. Nach 16 Stunden bei Raumtemperatur wird das Methanol in Vakuum entfernt, der Rückstand mit Wasser verdünnt und mit Essigester extrahiert. Die wäßrige Phase wird mit wäßriger 2 N HCl angesäuert und mit Essigester extrahiert. Nach Trocknen der organischen Phase über $MgSO_4$ wird diese eingeengt. Das Rohprodukt wird an Kieselgel chromatographiert (Laufmittel: Methylenchlorid/Methanol (15:1). Man erhält 0,66 g farbloses Öl.
$[\alpha]_D^{23}$ = -68,2° (c = 1, $CH_2Cl_2$)
$^1$H-NMR ($CDCl_3$):
δ = 7,8 (s, 1H); 7,2 (s, 5H); 7,05-6,6 (m, 1H); 5,9-5,5 (m, 1H); 4,9-4,1 (m, 1H); 3,7-3,2 (m, 2H); 2,8-2,4 (m, 2H); 2,4-1,6 (m, 8H) ppm.

Beispiel 4

3-[N-(4-Phenylbutyryl)-pyrrolidin-2-S-yl]-acrylsäure-pyrrolidid

1 g 3-[N-(4-Phenylbutyryl)-pyrrolidin-2-S-yl]-acrylsäure wird in 20 ml DMF mit 0,53 g 1-Hydroxybenztriazol, 0,4 g N-Ethylmorpholin und 0,23 g Pyrrolidin versetzt. 0,72 g Dicyclohexylcarbodiimid werden zugegeben und die Lösung 2 Stunden bei Raumtemperatur gerührt. Der ausgefallene Dicyclohexylharnstoff wird abgesaugt und das Lösungsmittel im Vakuum entfernt. Der Rückstand wird in Essigester aufgenommen und nacheinander mit wäßriger 1N $NaHCO_3$-Lösung, wäßriger 10 %iger Citronensäure, wäßriger 1 N $NaHCO_3$-Lösung und gesättigter wäßriger Kochsalzlösung gewaschen, über $Na_2SO_4$ getrocknet und eingeengt. Chromatographie an Kieselgel ergibt 0,24 g der Titelverbindung als Öl.
$[\alpha]_D^{21}$ = -46,9° (c = 0,104; $CH_2Cl_2$)
$^1$H-NMR ($CDCl_3$):
δ = 7,25 (s, 5H), 7,1-6,55 (m,1H); 6,4-5,9 (m, 1H), 5,0-4,6 (m, 1H); 3,8-3,3 (m,6H); 3,0-2,5 (m, 2H); 2,5-1,5 (m, 12H) ppm.

Beispiel 5

3-[N-(4-Phenylbutyryl)-pyrrolidin-2-S-yl]-acrylsäure-N-propylamid

Nach dem in Beispiel 4 angegebenen Verfahren wird 1 g 3-[N-(4-Phenylbutyryl)pyrrolidin-2S-yl]-acrylsäure mit 0,205 g N-Propylamin umgesetzt. Man erhält 0,65 g der Titelverbindung als Öl nach Chromatographie an $SiO_2$ mit Methylenchlorid/Methanol (20:1).
$[\alpha]_D^{21}$ = -60,2° (c = 0,264, $CH_2Cl_2$)
$^1$H-NMR ($CDCl_3$)
δ = 7,2 (s, 5H); 7,0-6,5 (m, 1H); 6,0-5,5 (m, 1H); 5,0-4,3 (m, 1H); 3,7-3,05 (m, 4H); 2,8-2,5 (m, 2H); 2,5-1,3 (m, 10H); 0,93 (t, 3H) ppm.

Beispiel 6

3-[N-(4-Phenylbutyryl)-pyrrolidin-2-S-yl]-acrylsäure-N-benzylamid

Nach dem in Beispiel 4 angegebenen Verfahren wird 1 g 3-[N-(4-Phenylbutyryl)-pyrrolidin-2-S-yl]-acrylsäure mit 0,37 g Benzylamin ungesetzt. Nach Chromatographie an Kieselgel mit Methylenchlorid/Methanol (25:1) als Laufmittel erhält man 0,8 g der Titelverbindung; Schmp. 75-76°C (aus

Essigester/Diisopropylether),
$[\alpha]_D^{21}$ = -50,8° (c = 0,206, $CH_2Cl_2$).
$^1$H-NMR ($CDCl_3$):
δ = 7,3-7,1 (m, 10H); 7,0-6,5 (m, 1H); 6,0-5,5 (m, 1H); 5,0-4,3 (m, 1H); 4,53-4,43 (2s, 2H); 3,6-3,2 (m, 2H); 2,9-2,5 (m, 2H); 2,5-1,2 (m, 8H) ppm.

Beispiel 7

3-[N-(4-Phenylbutyryl)-pyrrolidin-2,S-yl]-acrylsäure-(4-methyl-piperazid) Hydrochlorid

Nach der in Beispiel 4 angegebenen Vorschrift wird 1 g 3-[N-4-Phenylbutyryl)-pyrrolidin-2-S-yl]-acrylsäure mit 0,39 g N-Methylpiperazin umgesetzt. Die durch Chromatographie an Kieselgel ($CH_2Cl_2$/Methanol = 10:1) gereinigte freie Base der Titelverbindung wird in ethanolischer Salzsäure (2,5 N) aufgenommen und eingeengt. Man erhält das Hydrochlorid als Harz.
$[\alpha]_D^{24}$ = -34,25° (c = 0,216, Methanol).
$^1$H-NMR ($CDCl_3$):
δ = 7,2 (s, 5H); 7,0-6,0 (m, 2H); 4,8-4,3 (m, 1H); 3,9-3,3 (m, 6H)I 2,9-1,4 (m, 14H); 2,27 (s, 3H) ppm.

Beispiel 8

3-[N-(4-Phenylbutyryl)-pyrrolidin-2-S-yl]-acrylsäure-N-[2-(3,4-dimethoxyphenyl)ethyl[amid

Nach der in Beispiel 4 angegebenen Vorschrift wird 1 g 3-[N-(4-Phenylbutyryl)-pyrrolidin-2-S-yl]-acrylsäure mit 0,63 g Homoveratrylamin umgesetzt. Nach Chromatographie an Kieselgel mit Methylenchlorid/Methanol (25:1) erhält man 1,1 g der Titelverbindung als farblose Kristalle, Schmp. 89-90°C (aus Essigester/Diisopropylether)
$[\alpha]_D^{21}$ : -44° (c = 0,28, $CH_2Cl_2$)
$^1$H-NMR ($CDCl_2$) :
δ = 7,3-7,0 (m, 5H); 6,8-6,6 (m, 3H); 7,0-6,4 (m, 1H); 6,0-5,4 (m, 1H); 4,8-4,3 (m, 1H); 3,86 (s, 6H); 4,0-3,2 (m, 4H); 3,0-2,5 (m, 4H); 2,5-1,6 (m, 8H) ppm.

Beispiel 9

3-[N-(4-Phenylbutyryl)-pyrrolidin-2-S-yl]-acrylsäure-N-(4-phenyl)-piperazid

Nach der in Beispiel 4 angegebenen Vorschrift wird 1 g 3-[N-(4-Phenylbutyryl)-pyrrolidin-2-S-yl]-acrylsäure mit 0,56 g Phenylpiperazin umgesetzt. Nach Chromatographie an Kieselgel mit Methylenchlorid/Methanol (21:1) erhält man 1,1 g der Titelverbindung als Harz.
$[\alpha]_D^{24}$ = -33,5° (c = 0,31/$CH_3OH$)
$^1$H-NMR ($CDCl_3$) :
δ = 7,3-6,8 (m, 10H); 7,0-6,0 (m, 2H); 4,9-4,3 (m, 1H); 4,0-3,3 (m, 6H); 3,3-3,0 (m, 4H); 2,8-2,5 (m, 2H); 2,5-1,7 (m, 8H) ppm.

**Ansprüche**

1. Verwendung der Formel I

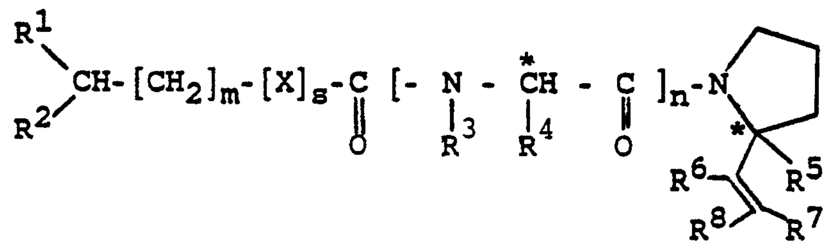

(I)

in welcher
$R^1$Wasserstoff; ($C_1$-$C_6$)-Alkyl oder ($C_6$-$C_{12}$)-Aryl, das gegebenenfalls durch einen, zwei oder drei gleiche oder verschiedene Reste aus der Reihe ($C_1$-$C_4$)-Alkyl, ($C_1$-$C_4$)-Alkoxy, Halogen, Nitro, Hydroxy, Amino, (-($C_1$-$C_4$)-Alkylamino und Di-($C_1$-$C_4$)-alkylamino, oder ein ($C_1$ oder $C_2$)-Alkylendioxy substituiert ist, bedeutet;
$R^2$ Wasserstoff; ($C_1$-$C_6$)-Alkyl; ($C_6$-$C_{12}$)-Aryl; ($C_6$-$C_{12}$)-Aryloxy; ($C_7$-$C_{13}$)-Aroyl; Hydroxy oder ($C_1$-$C_4$)-Alkoxy bedeutet, wobei Aryl, Aryloxy und Aroyl jeweils gegebenenfalls durch einen, zwei oder drei gleiche oder verschiedene Reste aus der Reihe ($C_1$-$C_4$)-Alkyl, ($C_1$-$C_4$)-Alkoxy, Halogen, Nitro, Hydroxy, Amino, ($C_1$-$C_4$)-Alkylamino und Di-($C_1$-$C_4$)-alkylamino, oder ein ($C_1$ oder $C_2$)-Alkylendioxy substituiert ist, bedeutet; oder
$R^1$ und $R^2$ zusammen für Benzyliden stehen, worin der Phenylring gegebenenfalls durch einen, zwei oder drei gleiche oder verschiedene Reste aus der Reihe ($C_1$-$C_4$)-Alkyl, ($C_1$-$C_4$)-Alkoxy, Halogen, Nitro, Hydroxy, Amino, ($C_1$-$C_4$)-Alkylamino, und Di-($C_1$-$C_4$)-alkylamino oder ein ($C_1$ oder $C_2$)-Alkylendioxy substituiert ist;
$R^3$ Wasserstoff; ($C_1$-$C_6$)-Alkyl; ($C_6$-$C_{12}$)-Aryl-($C_1$-$C_6$)-alkyl; ($C_5$-$C_9$)-Cycloalkyl; 1-oder 2-Indanyl oder $\alpha$-oder $\beta$-1,2,3,4-Tetrahydronaphthyl bedeutet;
$R^4$ Wasserstoff; ($C_1$-$C_6$)-Alkyl, das gegebenenfalls durch Amino, ($C_1$-$C_6$)-Acylamino oder Benzoylamino monosubstituiert sein kann; ($C_2$-$C_6$)-Alkenyl; ($C_5$-$C_9$)-Cycloalkyl; ($C_5$-$C_9$)-Cycloalkenyl; ($C_5$-$C_7$)-Cycloalkyl-($C_1$-$C_4$)-alkyl; ($C_6$-$C_{12}$)-Aryl oder teilhydriertes ($C_6$-$C_{12}$)-Aryl, die jeweils durch einen, zwei oder drei gleiche oder verschiedene Reste aus der Reihe ($C_1$-$C_4$)-Alkyl, ($C_1$ oder $C_2$)-Alkoxy und Halogen substituiert sein können; ($C_6$-$C_{12}$)-Aryl-($C_1$-$C_4$)-alkyl oder ($C_7$-$C_{13}$)-Aroyl-($C_1$ oder $C_2$)-alkyl, die beide wie vorstehend definiert im Arylrest substituiert sein können; einen mono-bzw. bicyclischen Heterocyclen-Rest mit 5 bis 7 bzw. 8 bis 10 Ringatomen, wovon 1 bis 9 Ringatome Kohlenstoffatome und 1 bis 2 Ringatome Schwefel- oder Sauerstoffatome und/oder wovon 1 bis 4 Ringatome Stickstoffatome darstellen; oder, falls von vorstehenden Definitionen noch nicht umfaßt, die gegebenenfalls geschützte Seitenkette einer natürlich vorkommenden $\alpha$-Aminosäure bedeutet; oder
$R^3$ und $R^4$ zusammen für -$[CH_2]_p$-stehen, worin p = 3, 4 oder 5 ist und worin eine Methylengruppe durch S oder O ersetzt sein kann;
$R^5$ Wasserstoff; ($C_1$-$C_6$)-Alkyl; ($C_6$-$C_{12}$)-Aryl-($C_1$-$C_5$)-alkyl, das im Arylteil gegebenenfalls durch einen, zwei oder drei gleiche oder verschiedene Reste aus der Reihe ($C_1$-$C_4$)-Alkyl, ($C_1$-$C_4$)-Alkoxy, Halogen, Nitro, Hydroxy, Amino, ($C_1$-$C_4$)-Alkylamino und Di-($C_1$-$C_4$)-alkylamino oder ein ($C_1$ oder $C_2$)-Alkylendioxy substituiert ist, bedeutet;
$R^6$ Wasserstoff; ($C_1$-$C_6$)-Alkyl oder ($C_6$-$C_{12}$)-Aryl, das gegebenenfalls durch einen oder zwei gleiche oder verschiedene Reste aus der Reihe ($C_1$-$C_4$)-Alkyl, ($C_1$ oder $C_2$)-Alkoxy und Halogen, oder ein ($C_1$ oder $C_2$)-Alkylendioxy substituiert ist, bedeutet;
$R^7$ Wasserstoff; ($C_1$-$C_6$)-Alkyl; Cyano; ($C_7$-$C_{13}$)-Aroyl, das gegebenenfalls durch einen oder zwei gleiche oder verschiedene Reste aus der Reihe ($C_1$-$C_4$)-Alkyl, ($C_1$ oder $C_2$)-Alkoxy, Halogen und Nitro, oder ein ($C_1$ oder $C_2$)-Alkylendioxy substituiert ist; ($C_1$-$C_6$)-Alkoxycarbonyl oder ($C_1$-$C_8$)-Alkanoyl bedeutet;
$R^8$ Cyano; ($C_7$-$C_{13}$)-Aroyl oder ($C_6$-$C_{12}$)-Aryl-($C_2$-$C_4$)-alkanoyl bedeutet, wobei jeweils Aryl durch einen, zwei oder drei gleiche oder verschiedene Reste aus der Reihe ($C_1$-$C_4$)-Alkyl, ($C_1$-$C_4$)-Alkoxy, Halogen, Nitro und Hydroxy oder ein ($C_1$ oder $C_2$)-Alkylendioxy substituiert sein kann; oder ($C_1$-$C_6$)-Alkanoyl; ($C_1$-$C_6$)-Alkoxycarbonyl; Benzyloxycarbonyl oder einen Rest der Formel

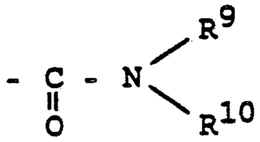

bedeutet;
$R^9$ und $R^{10}$ gleich oder verschieden sind und Wasserstoff; $(C_1-C_8)$-Alkyl; $(C_6-C_{12})$-Aryl oder $(C_6-C_{12})$-Aryl-$(C_1-C_8)$-alkyl, worin jeweils Aryl gegebenenfalls durch einen, zwei oder drei gleiche oder verschiedene Reste aus der Reihe $(C_1-C_4)$-Alkyl, $(C_1-C_4)$-Alkoxy, Halogen und Hydroxy, oder ein ($C_1$ oder $C_2$)-Alkylendioxy substituiert ist; oder $(C_5-C_9)$-Cycloalkyl bedeuten; oder
$R^9$ und $R^{10}$ zusammen mit dem diese tragenden N-Atom einen 5-bis 10-gliedrigen heterocyclischen Ring bilden, der als Ringatome 2-9 Kohlenstoffatome und gegebenenfalls 1 oder 2 weitere gleiche oder verschiedene Heteroatome aus der Reihe Sauerstoff, Schwefel und Stickstoff enthält und der gegebenenfalls durch einen Rest aus der Reihe $(C_1-C_8)$-Alkyl, Phenyl, Phenyl-$(C_1-C_4)$-alkyl, Benzoyl, Phenyl-$(C_2-C_4)$-alkanoyl, Furoyl, Pyridyl und Pyrimidinyl substituiert ist, worin Phenyl, Phenylalkyl, Benzoyl und Phenylalkanoyl ihrerseits jeweils im Phenylrest durch einen, zwei oder drei gleiche oder verschiedene Reste aus der Reihe $(C_1-C_4)$-Alkyl, $(C_1-C_4)$-Alkoxy, Halcgen, Hydroxy, Cyano und Nitro, oder ein ($C_1$ oder $C_2$)-Alkylendioxy substituiert sein können;
X Sauerstoff, Imino oder N-$(C_1-C_8)$-Alkylimino bedeutet;
m = 0, 1, 2, 3, 4 oder 5 ist;
n = 0, 1 oder 2 ist und
s = 0 oder 1 ist,
sowie deren physiologisch verträgliche Salze, falls solche gebildet werden können.

2. Verbindung der Formel I gemäß Anspruch 1, in welcher
$R^1$ Wasserstoff; $(C_1-C_4)$-Alkyl oder Phenyl, das gegebenenfalls durch einen oder zwei gleiche oder verschiedene Reste aus der Reihe Methyl, Ethyl, Methoxy, Fluor, Chlor, Brom, Nitro, Hydroxy, Amino, Methylamino und Dimethylamino, oder drei Methoxy, oder ein Methylendioxy substituiert ist, bedeutet;
$R^2$ Wasserstoff; Phenyl; Phenoxy; Benzoyl; Hydroxy oder Methoxy bedeutet, wobei Phenyl, Phenoxy und Benzoyl jeweils gegebenenfalls durch einen oder zwei gleiche oder verschiedene Reste aus der Reihe Methyl, Ethyl, Fluor, Chlor, Brom, Nitro, Hydroxy, Amino, Methylamino und Dimethylamino, oder ein Methylendioxy substituiert ist, oder
$R^1$ und $R^2$ zusammen für Benzyliden stehen, worin der Phenylrest gegebenenfalls durch einen oder zwei gleiche oder verschiedene Reste aus der Reihe Methyl, Ethyl, Methoxy, Fluor, Chlor, Brom und Hydroxy, drei Methoxy, oder ein Methylendioxy substituiert ist;
$R^3$ Wasserstoff; $(C_1-C_4)$-Alkyl; Benzyl; Phenethyl; Cyclopentyl; Cyclohexyl oder 2-Indanyl bedeutet;
$R^4$ Wasserstoff oder die gegebenenfalls geschützte Seitenkette einer natürlich vorkommenden α-Aminosäure bedeutet; oder
$R^3$ und $R^4$ zusammen für einen Rest $-[CH_2]_p$-stehen, der wie im Anspruch 1 definiert ist und worin p = 3 oder 4 ist;
$R^5$ Wasserstoff; Methyl; Ethyl; Benzyl oder Phenethyl, welche jeweils im Phenylrest gegebenenfalls durch einen, zwei oder drei gleiche oder verschiedene Reste aus der Reihe Methoxy, Fluor, Chlor und Brom, oder ein Methylendioxy substituiert sind, bedeutet;
$R^6$ Wasserstoff; $(C_1-C_4)$-Alkyl; Phenyl; Methoxyphenyl; Dimethoxyphenyl; Tolyl; Xylyl; Fluorphenyl; Chlorphenyl oder Methylendioxyphenyl bedeutet;
$R^7$ Wasserstoff; $(C_1-C_4)$-Alkyl; Cyano; Benzoyl, das gegebenenfalls durch einen oder zwei gleiche oder verschiedene Reste aus der Reihe Methyl, Methoxy, Fluor, Chlor, Brom und Nitro, oder ein Methylendioxy substituiert ist; $(C_1-C_4)$-Alkoxycarbonyl oder $(C_1-C_5)$-Alkanoyl bedeutet;
$R^8$ Cyano; Benzoyl, das gegebenenfalls durch einen oder zwei gleiche oder verschiedene Reste aus der Reihe Methyl, Methoxy, Fluor, Chlor und Brom, drei Methoxy oder ein Methylendioxy substituiert ist; $(C_1-C_4)$-Alkanoyl; $(C_1-C_4)$-Alkoxycarbonyl oder einen Rest der Formel

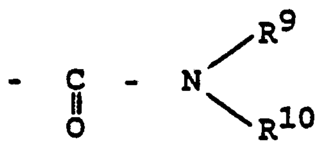

bedeutet;

$R^9$ und $R^{10}$ gleich oder verschieden sind und Wasserstoff; $(C_1$-$C_6)$-Alkyl; Phenyl oder Phenyl-$(C_1$-$C_4)$-alkyl, worin der Phenylrest jeweils durch einen oder zwei gleiche oder verschiedene Reste aus der Reihe Methyl, Ethyl, Methoxy, Fluor, Chlor, Brom und Hydroxy, drei Methoxy oder ein Methylendioxy substituiert sein kann; Cyclopentyl; Cyclohexyl oder Cycloheptyl bedeuten; oder $R^9$ und $R^{10}$ zusammen mit dem diese tragenden N-Atom einen 5-bis 7-gliedrigen heterocyclischen Ring bilden, der als Ringatome 2-6 Kohlenstoffatome und gegebenenfalls 1 oder 2 weitere gleiche oder verschiedene Heteroatome aus der Reihe Sauerstoff, Schwefel und Stickstoff enthält und der gegebenenfalls durch einen Rest aus der Reihe $(C_1$-$C_4)$-Alkyl, Phenyl, Phenyl-$(C_1$-$C_4)$-alkyl, Benzoyl, Furoyl, Pyridyl und Pyrimidinyl substituiert ist, worin Phenyl, Phenylalkyl und Benzoyl ihrerseits jeweils im Phenylrest durch einen oder zwei gleiche oder verschiedene Reste aus der Reihe Methyl, Methoxy, Fluor, Chlor, Brom, Cyano, Nitro und Hydroxy, drei Methoxy oder ein Methylendioxy substituiert sein können;

X Sauerstoff bedeutet;

m = 0, 1, 2, 3, 4 oder 5 ist;

n = 0 oder 1 ist und

s = 0 oder 1 ist,

sowie deren physiologisch verträgliche Salze.

3. Verwendung der Formel I gemäß Anspruch 1 oder 2, in welcher

$R^1$ Wasserstoff; Methyl; Ethyl, Propyl; Isopropyl; Phenyl; o-, m-oder p-Tolyl; o-, m-oder p-Chlorphenyl; o-, m-oder p-Fluorphenyl' o-, m-oder p-Methoxyphenyl oder 2,3-, 2,4-, 2,5-, 2,6-, 3,4-oder 3,5-Dimethoxyphenyl bedeutet;

$R^2$ Wasserstoff; Phenyl; o-, m-oder p-Tolyl; o-, m-oder p-Chlorphenyl; o-, m-oder p-Fluorphenyl; o-, m-oder p-Methoxyphenyl; 2,3-, 2,4-, 2,5-, 2,6-, 3,4 oder 3,5-Dimethoxyphenyl; Phenoxy; o-, m-oder p-Tolyloxy; o-, m-oder p-Chlorphenoxy; o-, m-oder p-Fluorphenyl; o-, m-oder p-Methoxyphenoxy; 2,3-, 2,4-, 2,5-, 2,6-, 3,4-oder 3,5-Dimethoxyphenoxy; Benzoyl; o-, m-oder p-Toluoyl; o-, m-oder p-Chlorbenzoyl; o-, m-oder p-Fluorbenzoyl; o-, m-oder p-Methoxybenzoyl; 2,3-, 2,4-, 2,5-, 2,6-, 3,4-oder 3,5-Dimethoxybenzoyl; Hydroxy oder Methoxy bedeutet; oder

$R^1$ und $R^2$ zusammen für Benzyliden; o-, m-oder p-Methylbenzyliden; o-, m-oder p-Methoxybenzyliden oder 2,3-, 2,4-, 2,5-, 2,6-3,4-oder 3,5-Dimethoxybenzyliden stehen;

$R^3$ Wasserstoff, Methyl oder Benzyl bedeutet;

$R^4$ Wasserstoff; Methyl; n-Propyl; Isopropyl; n-Butyl; Isobutyl; sec. Butyl oder Benzyl bedeutet; oder

$R^3$ und $R^4$ zusammen für $-[CH_2]_3$-stehen;

$R^5$ Wasserstoff oder Methyl bedeutet;

$R^6$ Wasserstoff oder Methyl bedeutet;

$R^7$ Wasserstoff; Methyl, Cyano; Benzoyl; o-, m-oder p-Methoxybenzoyl; o-, m-oder p-Chlorbenzoyl; 2,3-, 2,4-, 2,5-, 2,6-, 3,4-oder 3,5-Dimethoxybenzoyl; Acetyl; Methoxycarbonyl oder Ethoxycarbonyl bedeutet;

$R^8$ Cyano; Formyl; Benzoyl; o-, m-oder p-Methoxybenzoyl; o-, m-oder p-Chlorbenzoyl, 2,3-, 2,4-, 2,5-, 2,6-, 3,4-oder 3,5-Dimethoxybenzoyl; Acetyl; Methoxycarbonyl; Ethoxycarbonyl oder einen Rest der Formel

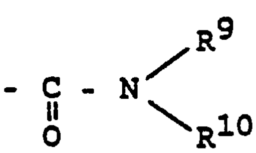

bedeutet;

$R^9$ und $R^{10}$ gleich oder verschieden sind und Wasserstoff; $(C_1$-$C_6)$-Alkyl; Phenyl oder Phenyl-$(C_1$-$C_4)$-alkyl, worin der Phenylrest jeweils durch einen oder zwei gleiche oder verschiedene Reste aus der Reihe Methyl, Methoxy, Fluor und Chlor, drei Methoxy, oder ein Methylendioxy substituiert sein kann; Cyclopentyl oder Cyclohexyl bedeutet; oder

$R^9$ und $R^{10}$ zusammen mit dem diese tragenden N-Atom einen 5-bis 7-gliedrigen heterocyclischen Ring bilden, der als Ringatome 2-6 Kohlenstoffatome und gegebenenfalls 1 oder 2 weitere gleiche oder verschiedene Heteroatome aus der Reihe Sauerstoff, Schwefel und Stickstoff enthält und der gegebenenfalls durch Methyl, Phenyl, Phenyl-($C_1$-$C_4$)-alkyl, Benzoyl, Pyridyl oder Pyrimidinyl monosubstituiert ist, worin Phenyl, Phenylalkyl und Benzoyl ihrerseits jeweils im Phenylrest durch ein oder zwei gleiche oder verschiedene Reste aus der Reihe Methyl, Methoxy, Fluor, Chlor, Cyano und Nitro oder drei Methoxy substituiert sein können;
X Sauerstoff bedeutet;
m = 0, 1, 2, 3, 4 oder 5 ist
n = 0 oder 1 ist und
s = 0 oder 1 ist,
sowie deren physiologisch verträgliche Salze.

4. Verbindung der Formel I gemäß einem der Ansprüche 1 bis 3, in welcher
$R^2$ Wasserstoff bedeutet;
$R^3$ und $R^4$ zusammen für $-[CH_2]_3$-stehen;
$R^5$ Wasserstoff bedeutet;
$R^6$ Wasserstoff bedeutet;
$R^7$ Wasserstoff bedeutet;
X Sauerstoff bedeutet;
m = 1, 2, 3 oder 4 ist;
n = 1 ist und
s = 0 oder 1 ist,
sowie deren physiologisch verträgliche Salze.

5. Verbindung der Formel I gemäß einem der Ansprüche 1 bis 3, in welcher
$R^5$ Wasserstoff bedeutet;
$R^6$ Wasserstoff bedeutet;
$R^7$ Wasserstoff bedeutet;
X Sauerstoff bedeutet;
m = 1, 2, 3 oder 4 ist;
n = 0 ist und
s = 0 oder 1 ist,
sowie deren physiologisch verträgliche Salze.

6. Verfahren zur Herstellung einer Verbindung der Formel I gemäß einem der Ansprüche 1 bis 5, dadurch gekennzeichnet, daß man
a) eine Verbindung der Formel II

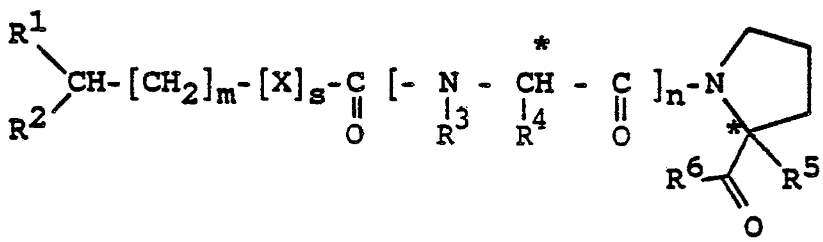

(II)

in welcher $R^1$, $R^2$, $R^3$, $R^4$, $R^5$ und $R^6$, X, m, n und s die gleiche Bedeutung wie in Formel I haben, in Gegenwart einer Base umsetzt mit einer Verbindung der Formel III,

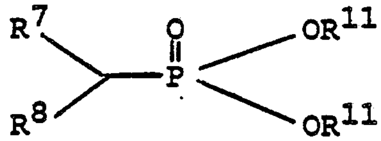

(III)

in welcher $R^7$ und $R^8$ die gleiche Bedeutung wie in Formel I haben und $R^{11}$ für ($C_1$-$C_4$)-Alkyl, Phenyl oder Phenyl-($C_1$-$C_4$)-alkyl steht; oder
b) eine Verbindung der unter (a) definierten Formel II umsetzt mit einer Verbindung der Formel IV

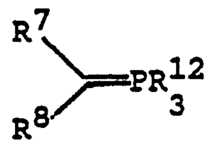 (IV)

in welcher $R^7$ und $R^8$ die gleiche Bedeutung wie in Formel I haben und $R^{12}$ für Phenyl, $(C_1\text{-}C_6)$-Alkylphenyl oder Di-$(C_1\text{-}C_6)$-alkylphenyl steht; oder
c) zur Herstellung einer Verbindung der Formel I, worin $R^8$ für einen Rest der Formel

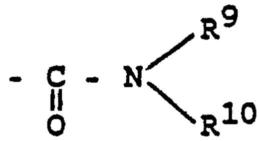

steht,
$c_1$) eine Verbindung der Formel I, in welcher $R^8$ $(C_1\text{-}C_6)$-Alkoxycarbonyl bedeutet und die übrigen Reste und Variablen wie oben definiert sind, umsetzt mit einer Verbindung der Formel V

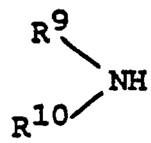 (V)

in welcher $R^9$ und $R^{10}$ die gleiche Bedeutung wie in Formel I haben; oder
$c_2$) eine Verbindung der Formel I, in welcher $R^8$ Carboxy bedeutet und die übrigen Reste und Variablen wie oben definiert sind, nach deren Umwandlung in ein aktiviertes Derivat mit einer Verbindung der unter ($c_1$) definierten Formel V umsetzt und
die nach (a), (b) oder (c) erhaltenen Verbindungen gegebenenfalls in ihre physiologisch verträglichen Salze überführt.

7. Verbindung gemäß einem der Ansprüche 1 bis 5 zur Anwendung als Heilmittel.

8. Verwendung gemäß einem der Ansprüche 1 bis 5 zur Anwendung als Prolyl-Endopeptidase-Hemmer.

9. Pharmazeutische Zubereitung, enthaltend eine Verbindung gemäß einem der Ansprüche 1 bis 5.

10. Verfahren zur Herstellung einer Zubereitung gemäß Anspruch 9, dadurch gekennzeichnet, daß man den Wirkstoff zusammen mit einem physiologisch annehmbaren Träger und gegebenenfalls weiteren Zusatz-, Hilfs-und/oder Konservierungsstoffen in eine geeignete Darreichungsform bringt.

Patentansprüche für die folgenden Vertragsstaaten: ES, GR

1. Verfahren zur Herstellung einer Verbindung der Formel I

**Verfahren zur Herstellung einer Verbindung der Formel I**

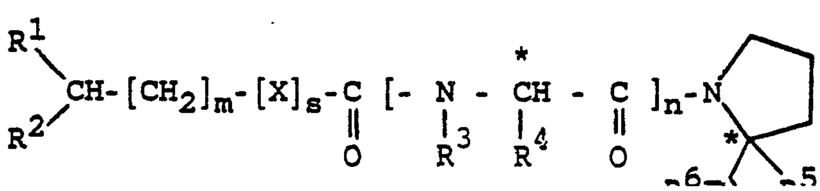 (I)

in welcher
$R^1$ Wasserstoff; $(C_1\text{-}C_6)$-Alkyl oder $(C_6\text{-}C_{12})$-Aryl, das gegebenenfalls durch einen, zwei oder drei gleiche

oder verschiedene Reste aus der Reihe ($C_1$-$C_4$)-Alkyl, ($C_1$-$C_4$)-Alkoxy, Halogen, Nitro, Hydroxy, Amino, (-($C_1$-$C_4$)-Alkylamino und Di-($C_1$-$C_4$)-alkylamino, oder ein ($C_1$ oder $C_2$)-Alkylendioxy substituiert ist, bedeutet;

$R^2$ Wasserstoff; ($C_1$-$C_6$)-Alkyl; ($C_6$-$C_{12}$)-Aryl; ($C_6$-$C_{12}$)-Aryloxy; ($C_7$-$C_{13}$)-Aroyl; Hydroxy oder ($C_1$-$C_4$)-Alkoxy bedeutet, wobei Aryl, Aryloxy und Aroyl jeweils gegebenenfalls durch einen, zwei oder drei gleiche oder verschiedene Reste aus der Reihe ($C_1$-$C_4$)-Alkyl, ($C_1$-$C_4$)-Alkoxy, Halogen, Nitro, Hydroxy, Amino, ($C_1$-$C_4$)-Alkylamino und Di-($C_1$-$C_4$)-alkykamino, oder ein ($C_1$ oder $C_2$)-Alkylendioxy substituiert ist, bedeutet; oder

$R^1$ und $R^2$ zusammen für Benzyliden stehen, worin der Phenylring gegebenenfalls durch einen, zwei oder drei gleiche oder verschiedene Reste aus der Reihe ($C_1$-$C_4$)-Alkyl, ($C_1$-$C_4$)-Alkoxy, Halogen, Nitro, Hydroxy, Amino, ($C_1$-$C_4$)-Alkylamino, und Di-($C_1$-$C_4$)-alkylamino oder ein ($C_1$ oder $C_2$)-Alkylendioxy substituiert ist;

$R^3$ Wasserstoff; ($C_1$-$C_6$)-Alkyl; ($C_6$-$C_{12}$)-Aryl-($C_1$-$C_6$)-alkyl; ($C_5$-$C_9$)-Cycloalkyl; 1-oder 2-Indanyl oder $\alpha$-oder $\beta$-1,2,3,4-Tetrahydronaphthyl bedeutet;

$R^4$ Wasserstoff; ($C_1$-$C_6$)-Alkyl, das gegebenenfalls durch Amino, ($C_1$-$C_6$)-Acylamino oder Benzoylamino monosubstituiert sein kann; ($C_2$-$C_6$)-Alkenyl; ($C_5$-$C_9$)-Cycloalkyl; ($C_5$-$C_9$)-Cycloalkenyl; ($C_5$-$C_7$)-Cycloalkyl-($C_1$-$C_4$)-alkyl; ($C_6$-$C_{12}$)-Aryl oder teilhydriertes ($C_6$-$C_{12}$)-Aryl, die jeweils durch einen, zwei oder drei gleiche oder verschiedene Reste aus der Reihe ($C_1$-$C_4$)-Alkyl, ($C_1$ oder $C_2$)-Alkoxy und Halogen substituiert sein können; ($C_6$-$C_{12}$)-Aryl-($C_1$-$C_4$)-alkyl oder ($C_7$-$C_{13}$)-Aroyl-($C_1$ oder $C_2$)-alkyl, die beide wie vorstehend definiert im Arylrest substituiert sein können; einen mono-bzw. bicyclischen Heterocyclen-Rest mit 5 bis 7 bzw. 8 bis 10 Ringatomen, wovon 1 bis 9 Ringatome Kohlenstoffatome und 1 bis 2 Ringatome Schwefel- oder Sauerstoffatome und/oder wovon 1 bis 4 Ringatome Stickstoffatome darstellen; oder, falls von vorstehenden Definition noch nicht umfaßt, die gegebenenfalls geschützte Seitenkette einer natürlich vorkommenden $\alpha$-Aminosäure bedeutet; oder

$R^3$ und $R^4$ zusammen für $-[CH_2]_p$-stehen, worin p = 3, 4 oder 5 ist und worin eine Methylengruppe durch S oder O ersetzt sein kann;

$R^5$ Wasserstoff; ($C_1$-$C_6$)-Alkyl; ($C_6$-$C_{12}$)-Aryl-($C_1$-$C_5$)-alkyl, das im Arylteil gegebenenfalls durch einen, zwei oder drei gleiche oder verschiedene Reste aus der Reihe ($C_1$-$C_4$)-Alkyl, ($C_1$-$C_4$)-Alkoxy, Halogen, Nitro, Hydroxy, Amino, ($C_1$-$C_4$)-Alkylamino und Di-($C_1$-$C_4$)-alkylamino oder ein ($C_1$ oder $C_2$)-Alkylendioxy substituiert ist, bedeutet;

$R^6$ Wasserstoff; ($C_1$-$C_6$)-Alkyl oder ($C_6$-$C_{12}$)-Aryl, das gegebenenfalls durch einen oder zwei gleiche oder verschiedene Reste aus der Reihe ($C_1$-$C_4$)-Alkyl, ($C_1$ oder $C_2$)-Alkoxy und Halogen, oder ein ($C_1$ oder $C_2$)-Alkylendioxy substituiert ist, bedeutet;

$R^7$ Wasserstoff; ($C_1$-$C_6$)-Alkyl; Cyano; ($C_7$-$C_{13}$)-Aroyl, das gegebenenfalls durch einen oder zwei gleiche oder verschiedene Reste aus der Reihe ($C_1$-$C_4$)-Alkyl, ($C_1$ oder $C_2$)-Alkoxy, Halogen und Nitro, oder ein ($C_1$ oder $C_2$)-Alkylendioxy substituiert ist; ($C_1$-$C_6$)-Alkoxycarbonyl oder ($C_1$-$C_8$)-Alkanoyl bedeutet;

$R^8$ Cyano; ($C_7$-$C_{13}$)-Aroyl oder ($C_6$-$C_{12}$)-Aryl-($C_2$-$C_4$)-alkanoyl bedeutet, wobei jeweils Aryl durch einen, zwei oder drei gleiche oder verschiedene Reste aus der Reihe ($C_1$-$C_4$)-Alkyl, ($C_1$-$C_4$)-Alkoxy, Halogen, Nitro und Hydroxy oder ein ($C_1$ oder $C_2$)-Alkylendioxy substituiert sein kann; oder ($C_1$-$C_6$)-Alkanoyl; ($C_1$-$C_6$)-Alkoxycarbonyl; Benzyloxycarbonyl oder einen Rest der Formel

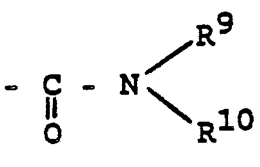

bedeutet;

$R^9$ und $R^{10}$ gleich oder verschieden sind und Wasserstoff; ($C_1$-$C_8$)-Alkyl; ($C_6$-$C_{12}$)-Aryl oder ($C_6$-$C_{12}$)-Aryl-($C_1$-$C_8$)-alkyl, worin jeweils Aryl gegebenenfalls durch einen, zwei oder drei gleiche oder verschiedene Reste aus der Reihe ($C_1$-$C_4$)-Alkyl, ($C_1$-$C_4$)-Alkoxy, Halogen und Hydroxy, oder ein ($C_1$ oder $C_2$)-Alkylendioxy substituiert ist; oder ($C_5$-$C_9$)-Cycloalkyl bedeuten; oder

$R^9$ und $R^{10}$ zusammen mit dem diese tragenden N-Atom einen 5-bis 10-gliedrigen heterocyclischen Ring bilden, der als Ringatome 2-9 Kohlenstoffatome und gegebenenfalls 1 oder 2 weitere gleiche oder verschiedene Heteroatome aus der Reihe Sauerstoff, Schwefel und Stickstoff enthält und der gegebenenfalls durch einen Rest aus der Reihe ($C_1$-$C_8$)-Alkyl, Phenyl, Phenyl-($C_1$-$C_4$)-alkyl, Benzoyl, Phenyl-($C_2$-$C_4$)-alkanoyl, Furoyl, Pyridyl und Pyrimidinyl substituiert ist, worin Phenyl, Phenylalkyl, Benzoyl und Phenylalkanoyl ihrerseits jeweils im Phenylrest durch einen, zwei oder drei gleiche oder verschiedene Reste aus der Reihe ($C_1$-$C_4$)-Alkyl, ($C_1$-$C_4$)-Alkoxy, Halogen, Hydroxy, Cyano und Nitro, oder ein ($C_1$ oder $C_2$)-Alkylendioxy substituiert sein können;

X Sauerstoff, Imino oder N-($C_1$-$C_8$)-Alkylamino bedeutet;
m = 0, 1, 2, 3, 4 oder 5 ist;
n = 0, 1 oder 2 ist und
s = 0 oder 1 ist,
sowie deren physiologisch verträgliche Salze, falls solche gebildet werden können, dadurch gekennzeichnet, daß man
a) eine Verbindung der Formel II

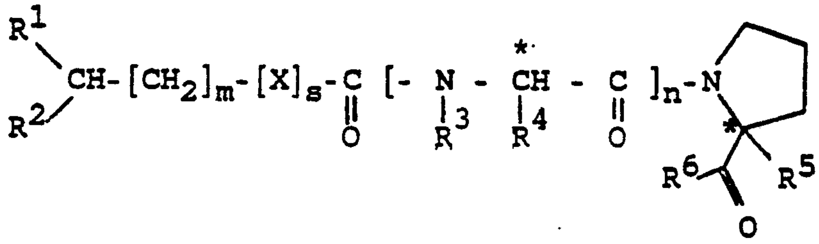
(II)

in welcher $R^1$, $R^2$, $R^3$, $R^4$, $R^5$ und $R^6$, X, m, n und s die gleiche Bedeutung wie in Formel I haben, in Gegenwart einer Base umsetzt mit einer Verbindung der Formel III,

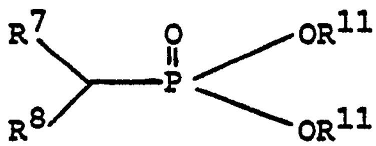
(III)

in welcher $R^7$ und $R^8$ die gleiche Bedeutung wie in Formel I haben und $R^{11}$ für ($C_1$-$C_4$)-Alkyl, Phenyl oder Phenyl-($C_1$-$C_4$)-alkyl steht; oder
b) eine Verbindung der unter (a) definierten Formel II umsetzt mit einer Verbindung der Formel IV

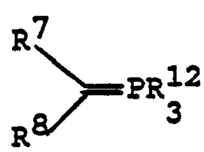
(IV)

in welcher $R^7$ und $R^8$ die gleiche Bedeutung wie in Formel I haben und $R^{12}$ für Phenyl, ($C_1$-$C_6$)-Alkylphenyl oder Di($C_1$-$C_6$)-alkylphenyl steht; oder
c) zur Herstellung einer Verbindung der Formel I, worin $R^8$ für einen Rest der Formel

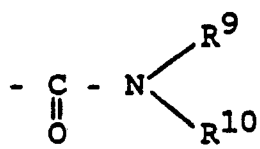

steht,
$c_1$) eine Verbindung der Formel I, in welcher $R^8$ ($C_1$-$C_6$)-Alkoxycarbonyl bedeutet und die übrigen Reste und Variablen wie oben definiert sind, umsetzt mit einer Verbindung der Formel V

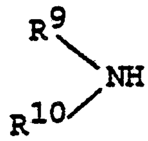

(V)

in welcher $R^9$ und $R^{10}$ die gleiche Bedeutung wie in Formel I haben; oder

$c_2$) eine Verbindung der Formel I, in welcher $R^8$ Carboxy bedeutet und die übrigen Reste und Variablen wie oben definiert sind, nach deren Umwandlung in ein aktiviertes Derivat mit einer Verbindung der unter ($c_1$) definierten Formel V umsetzt und

die nach (a), (b) oder (c) erhaltenen Verbindungen gegebenenfalls in ihre physiologisch verträglichen Salze überführt.

2. Verfahren gemäß Anspruch 1, dadurch gekennzeichnet, daß man eine Verbindung der Formel I herstellt, in welcher

$R^1$ Wasserstoff; ($C_1$-$C_4$)-Alkyl oder Phenyl, das gegebenenfalls durch einen oder zwei gleiche oder verschiedene Reste aus der Reihe Methyl, Ethyl, Methoxy, Fluor, Chlor, Brom, Nitro, Hydroxy, Amino, Methylamino und Dimethylamino, oder drei Methoxy, oder ein Methylendioxy substituiert ist, bedeutet;

$R^2$ Wasserstoff; Phenyl; Phenoxy; Benzoyl; Hydroxy oder Methoxy bedeutet, wobei Phenyl, Phenoxy und Benzoyl jeweils gegebenenfalls durch einen oder zwei gleiche oder verschiedene Reste aus der Reihe Methyl, Ethyl, Fluor, Chlor, Brom, Nitro, Hydroxy, Amino, Methylamino und Dimethylamino, oder ein Methylendioxy substituiert ist; oder

$R^1$ und $R^2$ zusammen für Benzyliden stehen, worin der Phenylrest gegebenenfalls durch einen oder zwei gleiche oder verschiedene Reste aus der Reihe Methyl, Ethyl, Methoxy, Fluor, Chlor, Brom und Hydroxy, drei Methoxy, oder ein Methylendioxy substituiert ist;

$R^3$ Wasserstoff; ($C_1$-$C_4$)-Alkyl; Benzyl; Phenethyl; Cyclopentyl; Cyclohexyl oder 2-Indanyl bedeutet;

$R^4$ Wasserstoff oder die gegebenenfalls geschützte Seitenkette einer natürlich vorkommenden α-Aminosäure bedeutet; oder

$R^3$ und $R^4$ zusammen für einen Rest -$[CH_2]_p$-stehen, der wie im Anspruch 1 definiert ist und worin p = 3 oder 4 ist;

$R^5$ Wasserstoff; Methyl; Ethyl; Benzyl oder Phenethyl, welche jeweils im Phenylrest gegebenenfalls durch einen, zwei oder drei gleiche oder verschiedene Reste aus der Reihe Methoxy, Fluor, Chlor und Brom, oder ein Methylendioxy substituiert sind, bedeutet;

$R^6$ Wasserstoff; ($C_1$-$C_4$)-Alkyl; Phenyl; Methoxyphenyl; Dimethoxyphenyl; Tolyl; Xylyl; Fluorphenyl; Chlorphenyl oder Methylendioxyphenyl bedeutet;

$R^7$ Wasserstoff; ($C_1$-$C_4$)-Alkyl; Cyano; Benzoyl, das gegebenenfalls durch einen oder zwei gleiche oder verschiedene Reste aus der Reihe Methyl, Methoxy, Fluor, Chlor, Brom und Nitro, oder ein Methylendioxy substituiert ist; ($C_1$-$C_4$)-Alkoxycarbonyl oder ($C_1$-$C_5$)-Alkanoyl bedeutet;

$R^8$ Cyano; Benzoyl, das gegebenenfalls durch einen oder zwei gleiche oder verschiedene Reste aus der Reihe Methyl, Methoxy, Fluor, Chlor und Brom, drei Methoxy oder ein Methylendioxy substituiert ist; ($C_1$-$C_4$)-Alkanoyl; ($C_1$-$C_4$)-Alkoxycarbonyl oder einen Rest der Formel

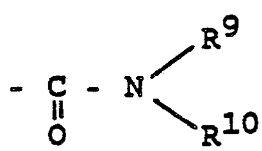

bedeutet;

$R^9$ und $R^{10}$ gleich oder verschieden sind und Wasserstoff; ($C_1$-$C_6$)-Alkyl; Phenyl oder Phenyl-($C_1$-$C_4$)-alkyl, worin der Phenylrest jeweils durch einen oder zwei gleiche oder verschiedene Reste aus der Reihe Methyl, Ethyl, Methoxy, Fluor, Chlor, Brom und Hydroxy, drei Methoxy oder ein Methylendioxy substituiert sein kann; Cyclopentyl; Cyclohexyl oder Cycloheptyl bedeuten; oder $R^9$ und $R^{10}$ zusammen mit dem diese tragenden N-Atom einen 5-bis 7-gliedrigen heterocyclischen Ring bilden, der als Ringatome 2-6 Kohlenstoffatome und gegebenenfalls 1 oder 2 weitere gleiche oder verschiedene Heteroatome aus der Reihe Sauerstoff, Schwefel und Stickstoff enthält und der gegebenenfalls durch einen Rest aus der Reihe ($C_1$-$C_4$)-Alkyl, Phenyl, Phenyl-($C_1$-$C_4$)-alkyl, Benzoyl, Furoyl, Pyridyl und Pyrimidinyl substituiert ist, worin Phenyl,

Phenylalkyl und Benzoyl ihrerseits jeweils im Phenylrest durch einen oder zwei gleiche oder verschiedene Reste aus der Reihe Methyl, Methocy, Fluor, Chlor, Brom, Cyano, Nitro und Hydroxy, drei Methoxy oder ein Methylendioxy substituiert sein können;
X Sauerstoff bedeutet;
m = 0, 1, 2, 3, 4 oder 5 ist;
n = 0 oder 1 ist und
s = 0 oder 1 ist,
sowie deren physiologisch verträgliche Salze.

3. Verfahren gemäß Anspruch 1 oder 2, dadurch gekennzeichnet, daß man eine Verbindung der Formel I herstellt, in welcher
$R^1$ Wasserstoff; Methyl; Ethyl; Propyl; Isopropyl; Phenyl; o-, m-oder p-Tolyl; o-, m-oder p-Chlorphenyl; o-, m-oder p-Fluorphenyl; o-, m-oder p-Methoxyphenyl oder 2,3-, 2,4-, 2,5-, 2,6-, 3,4-oder 3,5-Dimethoxyphenyl bedeutet;
$R^2$ Wasserstoff; Phenyl; o-, m-oder p-Tolyl; o-, m-oder p-Chlorphenyl; o-, m-oder p-Fluorphenyl; o-, m-oder p-Methoxyphenyl, 2,3-, 2,4-, 2,5-, 2,6-, 3,4-oder 3,5-Dimethoxyphenyl; Phenoxy; o-, m-oder p-Tolyloxy; o-, m-oder p-Chlorphenoxy; o-, m-oder p-Fluorphenoxy; o-, m-oder p-Methoxyphenoxy; 2,3-, 2,4-, 2,5-, 2,6-, 3,4-oder 3,5-Dimethoxyphenoxy; Benzoyl; o-, m-oder p-Toluoyl; o-, m-oder p-Chlorbenzoyl; o-, m-oder p-Fluorbenzoyl; o-, m-oder p-Methoxybenzoyl; 2,3-, 2,4-, 2,5-, 2,6-, 3,4-oder 3,5-Dimethoxybenzoyl; Hydroxy oder Methoxy bedeutet; oder
$R^1$ und $R^2$ zusammen für Benzyliden; o-, m-oder p-Methylbenzyliden; o-, m-oder p-Methoxybenzyliden oder 2,3-, 2,4-, 2,5-, 2,6-, 3,4-oder 3,5-Dimethoxybenzyliden stehen;
$R^3$ Wasserstoff, Methyl oder Benzyl bedeutet;
$R^4$ Wasserstoff; Methyl; n-Propyl; Isopropyl; n-Butyl; Isobutyl; sec. Butyl oder Benzyl bedeutet; oder
$R^3$ und $R^4$ zusammen für $-[CH_2]_3$-stehen;
$R^5$ Wasserstoff oder Methyl bedeutet;
$R^6$ Wasserstoff oder Methyl bedeutet;
$R^7$ Wasserstoff; Methyl; Cyano; Benzoyl; o-, m-oder p-Methoxybenzoyl; o-, m-oder p-Chlorbenzoyl; 2,3-, 2,4-. 2,5-, 2,6-, 3,4-oder 3,5-Dimethoxybenzoyl; Acetyl; Methoxycarbonyl oder Ethoxycarbonyl bedeutet;
$R^8$ Cyano; Formyl; Benzoyl; o-, m-oder p-Methoxybenzoyl; o-, m-oder p-Chlorbenzoyl, 2,3-, 2,4-, 2,5-, 2,6-, 3,4-oder 3,5-Dimethoxybenzoyl; Acetyl; Methoxycarbonyl; Ethoxycarbonyl oder einen Rest der Formel

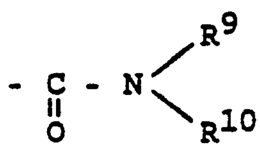

bedeutet;
$R^9$ und $R^{10}$ gleich oder verschieden sind und Wasserstoff; $(C_1-C_6)$-Alkyl; Phenyl oder Phenyl-$(C_1-C_4)$-alkyl, worin der Phenylrest jeweils durch einen oder zwei gleiche oder verschiedene Reste aus der Reihe Methyl, Methoxy, Fluor und Chlor, drei Methoxy, oder ein Methylendioxy substituiert sein kann; Cyclopentyl oder Cyclohexyl bedeutet; oder
$R^9$ und $R^{10}$ zusammen mit dem diese tragenden N-Atom einen 5-bis 7-gliedrigen heterocyclischen Ring bilden, der als Ringatome 2-6 Kohlenstoffatome und gegebenenfalls 1 oder 2 weitere gleiche oder verschiedene Heteroatome aus der Reihe Sauerstoff, Schwefel und Stickstoff enthält und der gegebenenfalls durch Methyl, Phenyl, Phenyl-$(C_1-C_4)$-alkyl, Benzoyl, Pyridyl oder Pyrimidinyl monosubstituiert ist, worin Phenyl, Phenylalkyl und Benzoyl ihrerseits jeweils im Phenylrest durch ein oder zwei gleiche oder verschiedene Reste aus der Reihe Methyl, Methoxy, Fluor, Chlor, Cyano und Nitro oder drei Methoxy substituiert sein können;
X Sauerstoff bedeutet;
m = 0, 1, 2, 3, 4 oder 5 ist
n = 0 oder 1 ist und
s = 0 oder 1 ist,
sowie deren physiologisch verträgliche Salze.

4. Verfahren gemäß einem der Ansprüche 1 bis 3, dadurch gekennzeichnet, daß man eine Verbindung der Formel I herstellt, in welcher
$R^2$ Wasserstoff bedeutet;

$R^3$ und $R^4$ zusammen für $-[CH_2]_3$-stehen;
$R^5$ Wasserstoff bedeutet;
$R^6$ Wasserstoff bedeutet;
$R^7$ Wasserstoff bedeutet;
X Sauerstoff bedeutet;
m = 1, 2, 3 oder 4 ist;
n = 1 ist und
s = 0 oder 1 ist,
sowie deren physiologisch verträgliche Salze.

5. Verfahren gemäß einem der Ansprüche 1 bis 3, dadurch gekennzeichnet, daß man eine Verbindung der Formel I herstellt, in welcher
$R^5$ Wasserstoff bedeutet;
$R^6$ Wasserstoff bedeutet;
$R^7$ Wasserstoff bedeutet;
X Sauerstoff bedeutet;
m = 1, 2, 3, oder 4 ist;
n = 0 ist und
s = 0 oder 1 ist,
sowie deren physiologisch verträgliche Salze.

6. Verfahren zur Herstellung einer pharmazeutischen Zubereitung enthaltend eine Verbindung gemäß einem der Ansprüche 1 bis 5, dadurch gekennzeichnet, daß man den Wirkstoff zusammen mit einem physiologisch annehmbaren Träger und gegebenenfalls weiteren Zusatz-, Hilfs-und/oder Konservierungsstoffen in eine geeignete Darreichungsform bringt.